Europäisches Patentamt

European Patent Office

Office européen des brevets

(19)

(11) Publication number : **0 605 370 A1**

(12) # EUROPEAN PATENT APPLICATION

(21) Application number : 93830522.4

(22) Date of filing : 23.12.93

(51) Int. Cl.[5] : **C12Q 1/18**

(30) Priority : **28.12.92 IT RM920932**

(43) Date of publication of application :
**06.07.94 Bulletin 94/27**

(84) Designated Contracting States :
**AT BE CH DE DK ES FR GB GR IE LI LU MC NL PT SE**

(71) Applicant : **CELLIFE BIOTECNOLOGIE PER LA VITA S.r.l.**
**Via XX Settembre 28/8**
**I-16121 Genova (IT)**

(72) Inventor : **Melioli, Giovanni, ISTITUTO NAZIONALE PER LA RICERCA SUL CANCRO,**
**Viale Benedetto XV, No. 10**
**I-16132 Genova (IT)**
Inventor : **Moretta, Lorenzo, ISTITUTO NAZIONALE PER LA RICERCA SUL CANCRO,**
**Viale Benedetto XV, No. 10**
**I-16132 Genova (IT)**

(74) Representative : **de Simone, Domenico et al**
**Ing. Barzanò & Zanardo Roma S.p.A.**
**Via Piemonte 26**
**I-00187 Roma (IT)**

(54) **Method to detect cytotoxic effectors.**

(57) A method to detect cytotoxic effectors, comprising the steps as follows : to incubate some target cells into a solution of said effector ; to separate the supernatant from the incubation mixture ; to detect at least one microsomial enzyme in this supernatant by means of an enzyme reaction ; to establish the ER (Experimental Release) value of said microsomial enzyme by deducting a control value from the obtained value, being the control value obtained by exposing said target cells to a solution, that is free from said cytotoxic effector.

FIG. 6

EP 0 605 370 A1

This invention relates to a method to detect microsomial enzymes following to lysis of target cells, that are exposed to cytotoxic effectors, to be used for laboratory assays.

Detection of death of target cells, due to given cytotoxic effectors, and the lysis thereof, is normally used as a cytotoxicity assay. This assay is used in a large extent in immunology procedures as well as in drug and cosmetic industries.

The detection of the NK (Natural Killer) cell activity, which may be present into the peripheral blood and is able to lysate target cells *in vitro,* is of special interest for immunology laboratories. The detection of NK activity is of great importance for research and diagnostics of patients affected by immunodeficiency or neoplasia diseases, or subjected to immunostimulating therapies, such as IL-2 (Interleukine 2), or interferon, or any immunosupressive therapy, such as chemiotherapy treatments.

Several cell lysis detection methods are well known; among them, it must be cited a method to measure the release of radioactive Cr isotopes, previously uptaken into target cells. Though this method is exact and reliable, the use thereof is restricted to only highly specialized laboratories, because of the presence of radioactive isotopes.

Another citotoxicity detecting method uses cytofluorometry techniques, therefore involving a substantial investment to purchase the equipment. Further, this method is slow, poorly reproducible and it cannot be automated.

Finally, a cell count on a microscope was carried out in the past on colored cells by some vital dye, that is obviously a very slow, automation unsuitable and poorly reproducible method.

The death of cultured cells will damage cell membrane, causing a leakage of the cytoplasm material into the culture medium.

The authors of the present invention found that it is possible to detect microsomial enzymes, normally located in the cytoplasm, in the culture medium of target cells, after incubation to cytotoxic effectors. Therefore, by dosing the amount of microsomial enzymes, it is possible to evaluate the lysis of target cells in a precise and reproducible way; moreover, the use of either radioactive material or specialized equipment is not required.

Further, the method of the invention can be easily automated and the calculation of results may be easily carried out by means of a data processing unit.

Finally, a number of assay samples can be collected and frozen, and the dosage thereof carried out later on.

Therefore it is an object of the invention a method to detect cytotoxic effectors, comprising the following steps:

- to incubate target cells into a solution comprising said effector;

- to separate the supernatant of the incubated mixture;
- to detect at least one microsomial enzyme into said supernatant by means of an enzymatic reaction; and
- to determine the experimental release (ER) of said microsomial enzyme by deducting a control value, obtained by exposing said target cells to a cytotoxic effector-free solution, from the value, as obtained as above.

According to a preferred embodiment of the invention, the cytotoxic effector comprises mononuclear cells of peripheral blood, preferably pre-treated *in vitro* with cytoxic promoter agents, preferably IL-2. Alternatively, the cytotoxic effector comprises natural, or recombinant, or synthetic materials to be used in drug or cosmetic industries.

According to the invention, said incubation is carried out for 2-6 hours at a temperature in the range from 25°C to 38°C, with an effector/target cell ratio ranging from 100:1 to 1,5:1, into an isotonic solution at pH ranging from 7.2 up to 7.5.

Furthermore, according to the invention, the supernatant separation from the incubation mixture is carried out by centrifuging this mixture.

According to another preferred embodiment of the invention, said microsomial enzyme is LDH (lactate deydrogenase) and said enzymatic reaction is performed by incubating with sodium pyruvate and $NADH^+$ (adenine-dinucleotide $H^+$), and by measuring the $NADH^+$ consumption, preferably by a 340nm reading on a spectrophotometer; most preferably being the sodium pyruvate concentration in the range from 0.4 up to 0.6 mg/ml, and $NADH^+$ concentration in the range from 3.0 up to 5.0 mg/ml.

The invention will be described by way of some illustrating, but not limiting examples, by reference to the annexed figures, wherein:

Fig.1 is a diagram showing the $NAHD^+$ consumption according to various LDH concentrations;

Fig.2 is a 340 nm absorbance curve with respect to the amount of the lysated cells through freezing and defrosting procedures;

Fig.3 shows six different experiments as indicated in the fig.2;

Fig.4 is a 340 nm absorbance curve with respect to the media, into which the cells were resuspended;

Fig.5 is a 340 nm absorbance curve with respect to used serum; and

Fig.6 shows the lysis percentages by using, as effectors, T lymphocytes from normal subjects or from neoplasia affected patients.

Example 1

PBMCs (Peripheral Blood Mononuclear Cells), isolated according to conventional techniques on discontinuous Ficoll-Paque gradient (Pharmacia), are used as cytoxicity effectors. Cells are collected at the gradient interface, washed three times with a RPMI serum-free medium (1640, Gibco) and then used. PBMCs are collected from healthy donors, as well as from patients suffering methastatic neoplasias.

PBMCs are treated with 1% of PHA (Phytohemoagglutinin) for two days and then incubated with 100 UI (International Units) of rIL-2 (recombinant IL-2, Proleukin®, Eurocetus). Cells are cultivated for 15 days. Highly cytotoxic lymphocytes are collected, separated from dead cells using a Ficoll-Paque gradient, washed with RPMI, counted and then used.

Target cells consist of the K562 line (ATCC N. CCL243), i.e., a microplasm-free, human erytroblastosis line. K562 cells are counted in a hemocytometer chamber before the assay and the dead cell pecentage calculated, using the Blue Tripan dye exclusion technique. Only cultures showing a dead cell amount less than 1% are used.

As to the macroscale test, effector cells, ranging from 1.000.000 to 15.000, are incubated with 20.000 K562 cells for 4 hours at 37°C into 0.5 ml of a 7% $CO_2$ solution of RPMI, together with antibiotics and glutamine. Test tubes are centrifuged for 5-7 minutes at 2.000 rpm, then 0.4 ml of supernatant are picked up from each test tube. Supernatants could be used immediately or frozen to -20°C.

As to the microscale test, effector cells, ranging from 500.000 to 15.000, were incubated with 10.000 K562 cells on microtitration trays with 96 V-shaped wells under the same conditions, as described for the macroscale test. Trays are centrifuged 5-7 minutes at 1,200 rpm; 0.2 mls of supernatant are picked up from each well and loaded on microtitration trays, having 96 flat bottom-type wells. The LDH detecting test is carried out immediately; as alternative, trays are frozen to -20°C.

As LHD enzyme is also present in the effector cells, the LHD spontaneous release must be evaluated. Accordingly, both the effectors and the target cells are separately incubated in parallel, as control.

LHD enzyme shall convert the sodium pyruvate in sodium lactate, in the presence of $NADH^+$. The LDH amount is proportional to the sodium lactate production, as well as to the $NADH^+$ concentration decrease, that could be detected by a 340 nm reading on a spectrophotometer. The optimized sodium pyruvate concentration is found to be 0.53 mg/ml, and the optimized $NADH^+$ concentration 4.0 mg/ml.

As to the macroscale test, the enzyme reaction is carried out directly using quartz microcuvettes to be read on a spectrophotometer, wherein a first reading is carried out before having added the enzyme substrates, and a second reading is carried out after having incubated for 7 minutes at 37°C. ER is calculated as the difference between the second and the first reading.

As to the microscale test, trays containing 0.2 ml of supernatant in each well are incubated in an ice bath, and 0.05 ml of a $NADH^+$/sodium pyruvate solution are added simultaneously to each well for 7 minutes at 37°C. The 340 nm absorbance is recorded before and after the reaction, using a multichannel photometer. The ER calculation is made as for the macroscale test.

All of tests are carried out in duplicate.

As control, ER values from target cell-free effectors (ESR), as well as the LDH total release (TTR) from K562 target cells, are measured after cell lysis, following a quick freezing-defrosting procedure. A further control could be carried out by using DAUDI cells (ATCC N. CCL213) as target cells, that are resistant against the lymphocyte cytotoxic action.

The specific LHD release (SpLR) could be calculated by the following equation:

$$SpLR_{(x:1)} = ER_{(x:1)} - ESR - TSR$$

wherein: 1.5 < x < 50

The lysis percentage could be calculated by the following equation:

$$Lysis\ percent = SpLR_{(x:1)}/(TTR - TSR) \times 100$$

wherein: 1.5 < x < 50

The LDH units could be calculated by the following equation:

$$LDHmU/ml = [SpLR_{(x:1)}per\ min. \times V_t]/[M.E.C. \times L.P. \times Vs]$$

wherein: UI = International Units; $SpLR_{(x:1)}$ = Experimental Release per minute; $V_t$ = total reaction volume; M.E.C. = $NADH^+$ Molar Consumption Factor, that is equivalent to 6.22 $cm^2$/uM at 340 nm; L.P. = ligth path length; and Vs = reaction volume.

The LUs (Lysis Units) could be calculated as the required effector amount to lysate 30% of 10.000 target cells, contained in 10 millions of cells globally.

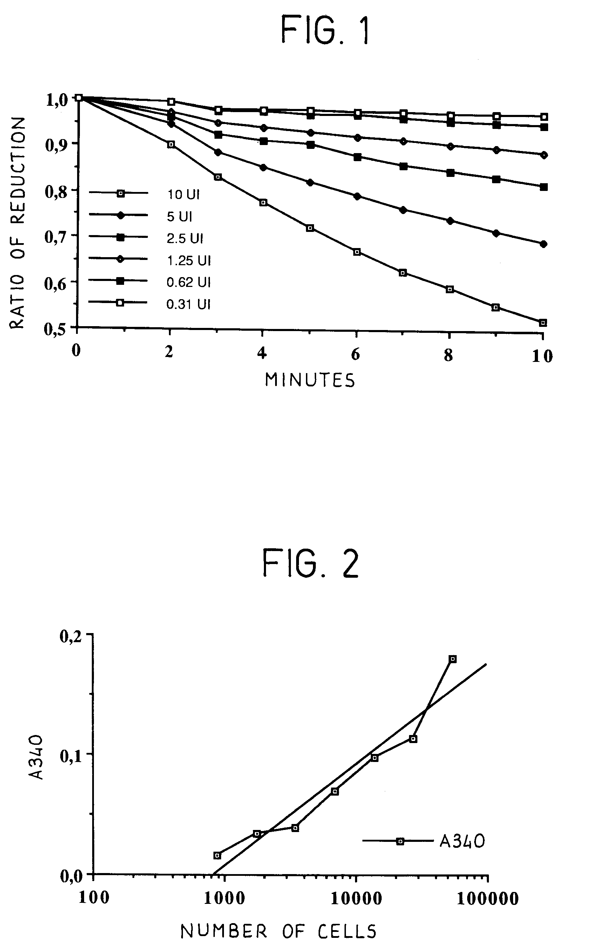

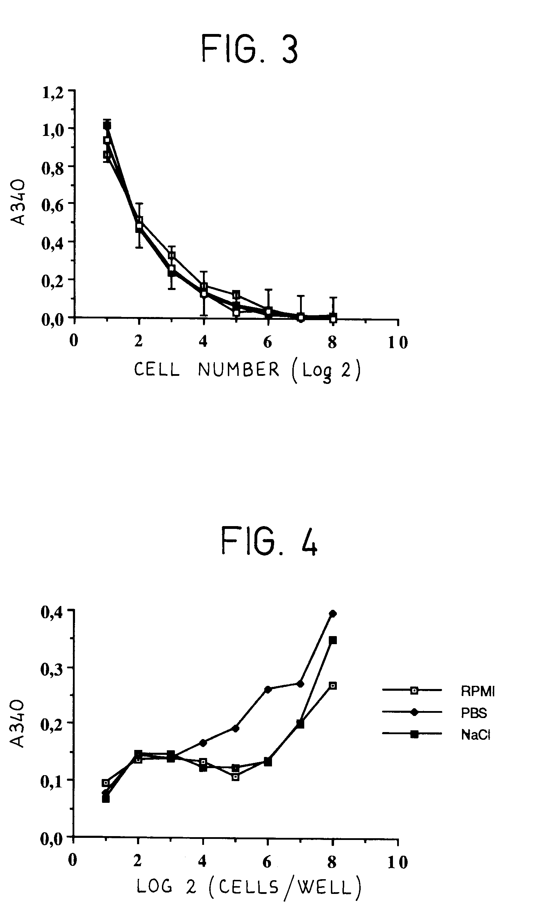

As shown in the fig.1, the $NADH^+$ consumption rate varies according to the LHD existing units. The assay sensitivity also is evaluated, by measuring the 340 nm absorbance in respect of lysated K562 cell amount by a freezing-defrosting procedure (see fig.2 and 3), that is found as being a linear function under the applied trial conditions.

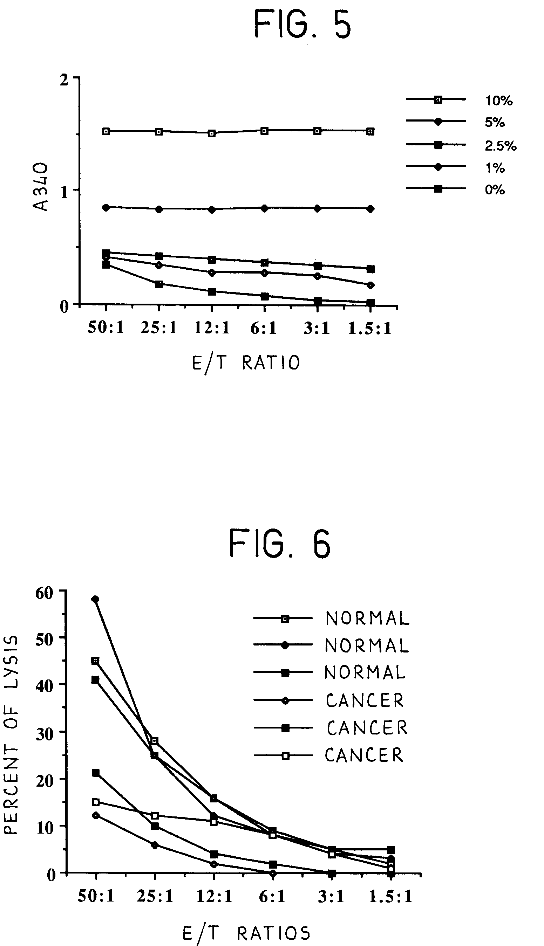

As shown in fig. 4 and 5, also the effects of the cell and serum resuspending media on the reaction development are analyzed.

The test allows to detect a difference among the lysis percentages, when the effectors are taken from healthy, or neoplasia affected patients, as shown in fig.4.

## Claims

1. A method to detect cytotoxic effectors from a sample, comprising the steps as follows:
   - to incubate target cells in a solution comprising said effector;

- to separate the supernatant of the incubated mixture;
- to detect at least one microsomial enzyme into said supernatant by means of an enzymatic reaction; and
- to determine the experimental release (ER) of said microsomial enzyme by deducting a control value, obtained by exposing said target cells to a cytotoxic effector-free solution, from the value, as obtained in the previous step.

2. A method to detect cytotoxic effectors according to claim 1, wherein said cytotoxic effector comprises mononuclear cells of human peripheral blood.

3. A method to detect cytotoxic effectors according to claim 2, wherein said peripheral blood mononuclear cells are pre-treated *in vitro* with cytoxic promoter agents.

4. A method to detect cytotoxic effectors according to claim 3, wherein said cytoxic promoter agent comprises IL-2.

5. A method to detect cytotoxic effectors according to claim 3, wherein said sample comprises natural or recombinant or synthetic material to be used in a drug or cosmetic manufacturing process.

6. A method to detect cytotoxic effectors according to any of previous claims, wherein said incubation is carried out with an effector/target cell ratio ranging from 100:1 to 1,5:1.

7. A method to detect cytotoxic effectors according to claim 6, wherein said incubation is carried out for 2-6 hours at a temperature ranging from 25°C and 38°C, in an isotonic solution at a pH ranging from 7.2 up to 7.5.

8. A method to detect cytotoxic effectors according to any of previous claims, wherein said supernatant separation from the incubation mixture is carried out by a centrifuging procedure.

9. A method to detect cytotoxic effectors according to claim 8, wherein said microsomial enzyme is LDH and the ER measurement thereof is carried out by means of an incubation in sodium pyruvate and $NADH^+$ and a measurement of said $NADH^+$ consumption.

10. A method to detect cytotoxic effectors according to the claim 9, wherein said $NADH^+$ consumption measurement is carried out by a 340 nm reading on a spectrophotometer.

11. A method to detect cytotoxic effectors according to claims 9 or 10, wherein the concentration of said sodium pyruvate ranges from 0.4 and 0.6 mg/ml, and the concentration of said $NADH^+$ ranges from 3.0 and 5.0 mg/ml.

# FIG. 1

# FIG. 2

# FIG. 3

# FIG. 4

# FIG. 5

# FIG. 6

European Patent Office

**EUROPEAN SEARCH REPORT**

Application Number

| DOCUMENTS CONSIDERED TO BE RELEVANT | | | EP 93830522.4 |
|---|---|---|---|
| Category | Citation of document with indication, where appropriate, of relevant passages | Relevant to claim | CLASSIFICATION OF THE APPLICATION (Int. Cl.5) |
| X | CHEMICAL ABSTRACTS, vol. 109, no. 13, September 26, 1988, Columbus, Ohio, USA CH. WANG et al. "LDH-release assay for natural cytotoxicity determination" page 459 * abstract-no. 108 607m * & Zhongguo Mianyixue Zazhi 1988, 4(1), 44-6 ---- | 1,2,9 | C 12 Q 1/18 |
| | | | TECHNICAL FIELDS SEARCHED (Int. Cl.5) |
| | | | C 12 Q |

The present search report has been drawn up for all claims

| Place of search | Date of completion of the search | Examiner |
|---|---|---|
| VIENNA | 28-03-1994 | SCHNASS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document